# EUROPEAN PATENT APPLICATION

(11) **EP 4 260 841 A1**
(43) Date of publication of application: **18.10.2023**
(21) Application number: 22305552.6
(22) Date of filing: 14.04.2022
(51) Int. Cl.: A61K 8/14, A61K 8/34, A61K 8/44, A61K 8/67, A61K 8/68, A61K 8/81, A61Q 19/00, A61Q 19/08

(54) **DELIVERY SYSTEM OF ACTIVE AGENTS VIA OSMOTIC GRADIENT**

(71) Applicant: Inayatbdv Ltd., London W1G 0JR (GB)
(72) Inventor: VALI, Shawana, London, W1G 0JR (GB)
(74) Representative: Cabinet Le Guen Maillet

(57) **Abstract**

The invention relates to a new delivery system of active agents and to topical compositions for application to human skin and to their use in maintaining or improving the condition and appearance of skin.

In particular, the invention relates to a composition, characterised in that it is provided in the form of an aqueous dispersion of spherules composed of at least one lipids bilayer encapsulating aqueous phase comprising at least one active agent, the lipids bilayer being composed of a mixture of liposoluble compounds comprising at least one (glycerylamidoethyl methacrylate and stearyl methacrylate) copolymer and at least one type III Ceramide and eventually at least one other liposoluble compound.

## Description

### FIELD OF THE INVENTION

This invention relates to a new delivery system of active agents and to topical compositions for application to human skin and to their use in maintaining or improving the condition and appearance of skin.

### BACKGROUND OF THE INVENTION

Skin is the largest organ of the body and is made up of three layers, the epidermis, dermis, and the hypodermis.

Skin is subject to alterations through the normal ageing process, environmental factors like air pollution, digital pollution or ultraviolets, or through dermatological disorders or diseases like acne, atopic dermatitis, or psoriasis, or can be weakened after dermatological or medical-esthetical treatments. In these conditions, skin homeostasis, in particular epidermis homeostasis, is impaired.

The homeostasis of the skin results from a balance between the processes of proliferation and of differentiation of the skin cells. This balance is regulated by many factors and intracellular signalling pathways. Homeostasis leads to the maintenance of the thickness of the skin and of its mechanical properties.

Impairment of homeostasis, in particular of dermal or epidermal homeostasis, leads to the loss of cells turnover, the decreasing of the barrier function and the skin thickness, the appearance of wrinkles.

Since many years the demand for products for improving the appearance and condition of skin has grown. Consumer's expectations are, for example, for products that keep the normal condition of the skin and that maintain its homeostasis, for products that treat or delay the visible signs of ageing such as wrinkles, fine lines, hyperpigmentation and age spots, loss of skin lightening, or for products that treat sensitive and dry skin.

The efficient and specific delivery of active agents is a critical challenge in any cosmetic or dermatological treatments.

A major difficulty to obtain effective cosmetic or dermatologic products is to ensure the active ingredients it contains can penetrate the skin and to reach its site of action which can be the epidermis or the dermis. The most important difficulty is that the stratum corneum forms an impermeable barrier that is difficult to pass for any external compound. Moreover, the skin is made up of variations of lipophilic and hydrophilic structures and the active ingredient must be adapted to this variation. Finally, the skin has an enzymatic system which can degrade the active ingredient.

Different ways of cutaneous penetration are available:
The transcellular route: the active agents penetrate the skin and diffuse from cell to cell.

This route is that of hydrophilic molecules.

The intercellular route: the active agents penetrate the skin and diffuse through the space between the cells, firstly in the inter-lipidic cement and secondly in the interstitial fluid. This route is that of lipophilic or amphiphilic substances.

The route through the skin appendages: the active agents penetrate the skin via the eccrine glands (trans glandular route) or via the hair follicles (trans follicular route) to diffuse into the reticular dermis. This route is used by lipophilic molecules.

The transcellular and intercellular routes are known as trans epidermal routes.

These different penetration routes all obey a passive mechanism defined according to the known Fick's law.

Many factors can influence the penetration of the active agents through the skin's layer. The most important of these factors is the nature of the vectors used and the hydration state of the skin.

Vectors are delivery systems usually used to improve the bioavailability of actives agents. They help to direct active agents to their site of action and to protect them against enzymatic degradation.

Choosing an appropriate delivery system can then produce optimal penetration by focusing and enhancing active passage through a desired penetration pathway. Liposomes are the most common vectors used in actives delivery in the cosmetic and dermatology field. Liposomes are spherical vesicles, or spherules, consisting of one or more lipid bilayers enclosing an aqueous compartment. Bilayers can be made of single amphiphilic lipids or different lipids. They vary in their dimensions, compositions of lipids, charge, number of layers and flexibility. Liposomes are used as carriers for both lipophilic and hydrophilic actives. Hydrophilic actives are entrapped in the aqueous compartment, while lipophilic actives are intercalated within the lipid bilayer. They are supposed to be able to disorder the intercellular lipid matrix for using the intercellular penetration route. Moreover, because of their bilayer structure, which is like the skin, cell membranes liposomes can combine with membranes lipids and release actives into cells. Numerous lipid compositions and preparation methods for liposomes exist, which affect several liposomal characteristics, such as size, penetration, and encapsulation effectiveness.

As previously mentioned, the hydration state of the skin is a key factor for improving skin penetration. Indeed, the osmotic gradient resulting from the difference in the total water concentrations between the skin surface and the epidermis provides attracting force for the lipidic vesicles. It has been shown that when the skin surface is occluded by occlusive patch, the osmotic gradient is diminished or disappears, and penetration of vesicles is severely decreased. Moreover, it has been shown that the permeability of an hydrated skin is superior than the permeability of a dehydrated skin, thus favouriting the penetration through the osmotic gradient.

So, depending on vesicles nature and composition and on hydration state of the skin, the success of the delivery of active agents to their site of action into the skin will vary.

### SUMMARY OF THE INVENTION

It is an object of the present invention to propose cosmetic and dermatologic topical compositions having improved skin penetration of actives agents' capacity.

Another object of the present invention is to propose cosmetic and dermatologic topical compositions having good hydration and skin care capacities.

Another object of the present invention is to propose a cosmetic process of a healthy skin consisting of applying to the healthy skin a cosmetic composition according to the invention.

Another object of the present invention is to propose uses of a dermatologic composition according to the invention to treat the skin.

Another object of the present invention is to propose a new delivery system for active agents into the skin.

### DETAILED DESCRIPTION OF THE INVENTION

In the context of the present invention, the term "spherule" encompasses vectors like vesicles formed by lipid layers encapsuling aqueous compartment or phase.

In the context of the present invention, the term "delivery system" designs vector used to conduct one or more active agents to their site of action into the skin.

In the context of the present invention, "healthy skin" designs an area of the skin, preferentially the visage, the neck, or the hands, which is considered "not pathologic" by a dermatology, that is which doesn't present infection, inflammation, cicatrix, cutaneous diseases like candida, eczema, dermatitis or wound.

In the context of the present invention, "a process for the cosmetic and non-therapeutic treatment of a healthy skin" designs a cosmetic process applied to a skin which is considered "not pathologic" by a dermatology, that is which doesn't present infection, inflammation, cicatrix, cutaneous diseases like acne, candida, eczema, dermatitis or wound.

So, in a first aspect, the invention concerns a cosmetic or dermatologic composition, characterised in that it is provided in the form of an aqueous dispersion of spherules composed of at least one lipids bilayer encapsulating aqueous phase comprising at least one active agent, the lipids bilayer being composed of a mixture of liposoluble compounds comprising at least one (glycerylamidoethyl methacrylate and stearyl methacrylate) copolymer and at least one type III Ceramide and eventually at least one other liposoluble compound.

Ceramides are class of lipids present in the intercellular lipids of stratum corneum. Ceramides are sphingolipids where fatty acid is connected to sphingosine via amide linkage. Several types of ceramides exist, and they are divided in classes of ceramides. Today, eleven classes of ceramides have been identified in the stratum corneum.

(Glycerylamidoethyl methacrylate and stearyl methacrylate) copolymer is a pseudo ceramide and a synthetic polymer that mimics the structure of natural type II ceramides, a constituent of the intercellular lipids of stratum corneum. It is commercially available for example under the name CERACUTE L^{®} (ROSSOW).

Ceramides III consist of a phytosphingosine backbone acylated with a saturated fatty acid. Ceramides III may be a natural ceramide, i.e. an extracted substance, a synthetic substance or can be obtained by a microbial fermentation method. Preferentially in the present invention, ceramides III have the same stereo-chemical configuration than ceramides III that exist in the human skin.

Such ceramides III are available as commercial products for example under the "Ceramide III" (EVONIK) containing N-stearoyl-phytosphingosine (type 3) as a component.

Ceramides III and (Glycerylamidoethyl methacrylate and stearyl methacrylate) copolymers are amphiphilic molecules that are used to form the lipid layers of the spherules of the composition of the invention.

It was surprisingly found that the composition of the invention, containing such specific spherules, improves the diffusion/penetration and the efficacy of the actives agents it contains.

The combination of the double ceramides according to the invention is very hygroscopic and completely hydrates the epidermis which renders the skin permeable to transdermal penetration of smaller molecules. As previously mentioned, the hydration state of the skin is a key factor to enhance skin penetration of lipid vesicles via the osmotic gradient. Then the spherules according to the invention are very effective to generate an osmotic gradient and to enhance skin penetration. The active agents it contains penetrate the stratum corneum due to its osmotic properties and are taken up by keratinocytes through specific transporters to restore global balance in the cells.

The aqueous solvent containing the spherules dispersion can be water or any other well-known aqueous solvent cosmetically acceptable.

According to one embodiment, the composition comprises between 1 to 30 %, by weight, of (glycerylamidoethyl methacrylate and stearyl methacrylate) copolymers and between 0,1 to 5% of at least one type III Ceramides, by weight relative to the total weight of the composition.

Advantageously, the composition comprises at least 1%, preferably at least 10%, more preferably at least 20%, more preferably at least 30%, of spherules, by weight relative to the total weight of the composition.

According to a first preferred embodiment, the composition of the invention comprises at least one another liposoluble compound which is bakuchiol or/and α-tocopheryl acetate, preferably a mixture of bakuchiol and α-tocopheryl acetate.

Bakuchiol and α-tocopheryl acetate are also integrated into lipids bilayer of the spherules. When they are used in the compositions of the invention they act at the same time as lipophile active agents and as builder of the spherules.

Bakuchiol is extracted and purified from Psoralea corylifolia (Babchi seeds).

α-tocopheryl acetate is the synthetic form of E vitamin.

Advantageously, the composition comprises between 1 to 10 % of bakuchiol and between 1 to 10 % of α-tocopheryl acetate, by weight relative to the total weight of the composition.

According to a second preferred embodiment, the aqueous phase encapsulated in the spherules is an aqueous solution comprising at least one active agent selected from Scenedesmus obliquus extract, tranexamic acid, L-glutathione, niacinamide, D-panthenol, tetrapeptide N-Palmitoyl-Gly-Gln-Pro-Arg, probiotic microorganisms of the specie lactobacillus plantarum, ascorbyl glucoside, seawater, niosomes of C vitamin or anyone of their combinations.

By "any one of their combinations" it must be understood that the composition can comprise one, two, three, four, five, six, seven, eight, nine or ten of the listed active agents.

According to a third preferred embodiment, the composition comprises tranexamic acid, L-glutathione, ascorbyl glucoside, niacinamide, seawater and D-panthenol as actives agents encapsulated in the aqueous phase of the spherules.

According to a fourth preferred embodiment, the composition comprises Scenedesmus obliquus extract, tranexamic acid, L-glutathione, niacinamide, D-panthenol, tetrapeptide N-Palmitoyl-Gly-Gln-Pro-Arg, probiotic microorganisms of the specie lactobacillus plantarum, ascorbyl glucoside, niosomes of C vitamin and seawater as actives agents encapsulated in the aqueous phase of the spherules.

All the listed active agents are commercially available.

Scenedesmus obliquus extract is obtained from Scenedesmus, a unicellular microalga from the Chlorophyceae class and from the scenedemacae family.

Tranexamic acid is a synthetic derivative of the amino acid lysine.

L-glutathione is a tripeptide comprised of three amino acids: glycine, cysteine and glutamic acid.

Niacinamide is B3 vitamin.

D-panthenol isa pro vitamin of D-pantothenic acid (B5 vitamin).

Tetrapeptide N-Palmitoyl-Gly-Gln-Pro-Arg (Palmitoyl Tetrapeptide-7) is a synthetic peptide comprised of four amino acids: glycine, proline, glutamine and arginine.

Lactobacillus plantarum is a Gram-positive lactic acid bacterium found in the gastro intestinal tract and commonly used in the food industry. Any known specie can be used but particularly preferred species are Lactobacillus plantarum (Heal 9) DSM 15312, Lactobacillus plantarum (Heal 19) DSM 15313, Lactobacillus plantarum (299v) DSM 9843.

Ascorbyl glucoside is a water soluble derivative of C vitamin.

Niosomes of C vitamin is a stabilized form of vitamin C derivative. According to this formulation, Ethyl Ascorbyl Ether (3-O-Ethyl Ascorbic Acid) is encapsulated into a niosomal ultra-deformable vesicle ( Niosoma Nio-VCS ).

According to this fourth embodiment, the composition comprises, by weight relative to the total weight of the composition:
- between 1 to 30 % of glycerylamidoethyl methacrylate and stearyl methacrylate copolymer,
- between 0,1 to 5% of at least one type III Ceramides,
- between 1 to 10 % of bakuchiol,
- between 1 to 10 % of α-tocopheryl acetate,
- between 1 to 15 % of tranexamic acid,
- between 1 to 15 % of ascorbyl glucoside,
- between 1 to 10 % of seawater,
- between 1 to 5 % of D-panthenol,
- between 1 to 5 % of probiotic microorganisms of the specie lactobacillus plantarum,
- between 1 to 5 % of L-glutathione,
- between 1 to 10 % tetrapeptide N-Palmitoyl-Gly-Gln-Pro-Arg,
- between 1 to 25 % of niacinamide,
- between 1 to 10 % of Scenedesmus obliquus extract,
- between 1 to 10% of niosomes of C vitamin.

Preferentially, the composition comprises, by weight relative to the total weight of the composition :
- between 15 to 25 % of glycerylamidoethyl methacrylate and stearyl methacrylate copolymer,
- between 0,1 to 0,5% of at least one type III Ceramides,
- between 4 to 8 % of bakuchiol,
- between 2 to 5 % of α-tocopheryl acetate,
- between 8 to 12 % of tranexamic acid,
- between 2 to 5 % of ascorbyl glucoside,
- between 4 to 8 % of seawater,
- between 2 to 5 % of D-panthenol,
- between 2 to 5 % of probiotic microorganisms of the specie lactobacillus plantarum,
- between 1 to 3 % of L-glutathione,
- between 4 to 8 % tetrapeptide N-Palmitoyl-Gly-Gln-Pro-Arg,
- between 15 to 22 % of niacinamide,
- between 4 to 8 % of Scenedesmus obliquus extract,
- between 4 to 6% of niosomes of C vitamin.

The composition of the invention takes the form of a gel or a liquid like a lotion.

As it will be demonstrated by the following examples, the composition according to this fourth embodiment is very efficient in cosmetic care of the healthy skin and in dermatologic treatment of damaged skin. The combination of actives renews, repairs, and enhances the skin energetic field on a cellular level, activates the skin's own cells to self-repair, and restores the homeostasis systems of the skin.

These particularly impressive effects result not only from the specific choice of actives but also from the combination of the selected active agents and the specific spherules formed by the ceramides which are very efficient to enhance penetration of the actives due to the osmotic gradient. As a result, the actives following the osmotic gradient penetrate the skin layers via the lipid cement and the interstitial fluid and are then taken up by the cells through specific transporters for, in particular, restoring and maintaining homeostasis in the cells.

In a second aspect, the invention concerns a process for preparing a composition according to the invention, characterised in that it comprises a first step of mixing glycerylamidoethyl methacrylate and stearyl methacrylate copolymer with type III Ceramides, and eventually bakuchiol or/and α-tocopheryl acetate, and a second step of adding an aqueous solvent comprising at least one active agent selected from Scenedesmus obliquus extract, tranexamic acid, L-glutathione, niacinamide, D-panthenol, tetrapeptide Palmitoyl-Gly-Gln-Pro-Arg, probiotic microorganism of the species lactobacillus plantarum, ascorbyl glucoside, niosomes of C vitamin, seawater or anyone of their combination.

The process according to the invention is very simple and consists in a first step to mixing together the lipophile compounds and in a second step to mixing the obtained lipophile mix with the hydrophile compounds.

More precisely, and in a preferred embodiment, the process according to the invention consist in:
- Mixing (glycerylamidoethyl methacrylate and stearyl methacrylate) copolymer, type III Ceramides, bakuchiol and α-tocopheryl acetate, to obtain a first mix,
- Mixing Scenedesmus obliquus extract, tranexamic acid, L-glutathione, niacinamide, D-panthenol, tetrapeptide Palmitoyl-Gly-Gln-Pro-Arg, probiotic microorganism of the species lactobacillus plantarum, ascorbyl glucoside, niosomes of C vitamin, seawater and an aqueous solvent, to obtain a second mix,
- Mixing the first and second mixes together.

A solution in the form of an emulsion of micelles or spherules is then obtained.

The composition according to the invention can be introduce as an active ingredient in other formulations.

So, in a third aspect, the invention concerns a cosmetic or dermatologic product, or composition, comprising a composition as previously described.

Such product could be a gel, a cream, an emulsion, a lotion, or any cosmetic or dermatologic galenic form adapted to the skin care.

Such product can comprise between 0,5 to 15 % of the composition previously described, weight of the product, between 0,5 to 15 % of a composition as defined in any one of claims 1 to 9.

So, the cosmetic or dermatologic product, will comprise :
- between 0.1 to 30 % of glycerylamidoethyl methacrylate and stearyl methacrylate copolymer,
- between 0.01 to 5 % of at least one type III Ceramides,
- between 0.5 to 10 % of bakuchiol,
- between 0.1 to 10 % of α-tocopheryl acetate,
- between 1 to 2.0 % of tranexamic acid, (réglementé à 2.0 % max)
- between 0.1 to 15 % of ascorbyl glucoside,
- between 0.5 to 10 % of seawater,
- between 0.1 to 5 % of D-panthenol,
- between 0.5 to 5 % of probiotic microorganisms of the specie lactobacillus plantarum,
- between 0.1 to 5 % of L-glutathione,
- between 0.0001 to 1 % tetrapeptide N-Palmitoyl-Gly-Gln-Pro-Arg,
- between 1 to 5 % of niacinamide,
- between 0.5 to 10 % of Scenedesmus obliquus extract,
- between 0.5 to 10 % of niosomes of C vitamin.

Such product may further comprises at least one other active agent and at least one component usually used in the cosmetic field and for example selected from thickeners, gelling agents, preservatives, emulsifiers, pH regulators and colorants.

In a fourth aspect, the invention concerns a process for the cosmetic, and non-therapeutic, treatment of a healthy skin, characterised in that it consists in applying to the healthy skin a sufficient amount of a composition as previously described.

According to this third aspect, the invention also concerns a non-therapeutic cosmetic use of a composition as previously described, for treating or preventing, on a healthy skin, skin ageing or photo-ageing, for moisturizing the skin, for reinforcing the lipid barrier of the epidermis, for restoring or maintaining the integrity of the stratum corneum, for maintaining, stimulating or restoring cutaneous homeostasis, in particular the epidermis homeostasis, for maintaining, stimulating, restoring or stimulating skin trophic activity, in particular for stimulating collagen and/or glycosaminoglycan and /or filaggrin synthesis, for stimulating the cellular energy metabolism, in particular for improving ATP synthesis and cell metabolism, for reducing Reactive Oxygen Species levels naturally occurring in skin cells, for maintaining, stimulating or restoring cell regeneration, for diminishing the appearance of wrinkles, for supporting natural barrier function, or for lightening, whitening or depigmenting the skin, in particular pigment spots or dark circles under the eyes, the cosmetic use being apply to an healthy skin.

In a fifth aspect, the invention concerns a composition as previously described for its use for preventing, reducing or treating skin inflammatory, for maintaining the skin microbiota balance, in particular in case of acne, for restoring skin after dermatological or chirurgical treatment or for restoring atopic skin, in particular for restoring cell regeneration, cell homeostasis, cellular energy metabolism and the integrity of the stratum corneum after such treatments or in atopic skin, for reducing oxidative stress in skin cells, in particular due to environmental factors like urban or digital pollution or UV, for reducing or treating skin dyschromia due to dermatologic diseases, for wound healing.

Finally, in a sixth aspect, the invention concerns a delivery system for active agents as such, characterized in that it comprises an aqueous dispersion of spherules composed of at least one lipids bilayer encapsulating aqueous phase, the lipids bilayer being composed of a mixture of liposoluble compounds comprising at least one (glycerylamidoethyl methacrylate and stearyl methacrylate) copolymer and at least one type III Ceramides and eventually at least one other liposoluble compound.

Advantageously, the delivery system comprises at least one another liposoluble compound which is bakuchiol or/and α-tocopheryl acetate, and preferably comprises bakuchiol and α-tocopheryl acetate.

According to the invention, the delivery system comprises at least 60 % of (glycerylamidoethyl methacrylate and stearyl methacrylate) copolymers, preferentially between 65 and 90%, and less than 40 % of type III Ceramides, preferably between 1 and 35 %, by weight relative to the total weight of the liposoluble compounds.

Advantageously, the delivery system comprises between 10 and 25 % of bakuchiol and between 5 and 15% of α-tocopheryl acetate.

### EXAMPLES

The present invention is illustrated in greater detail in the following examples.

### EXAMPLE 1: Formulation

A composition according to the invention is prepared from the ingredients listed in Table 1 below. In Table 1, the percentages are weight percentages.

**Table 1**

| **PHASE** | ***INCI*** | *% INCI* |
|---|---|---|
| **A** | **TYPE III CERAMIDE** | **0,336** |
| | CERAMIDE NP | 0,336 |
| **A** | **TYPE II CERAMIDE** | **20,202** |
| | GLYCERYLAMIDOETHYL | 1,010 |
| | METHACRYLA TE/STEARYL | |
| | METHACRYLATE COPOLYMER | |
| | GLYCERIN | 13,434 |
| | BUTYLENE GLYCOL | 5,757 |
| **C** | **NIACINAMIDE** | **20,202** |
| **C** | **TRANEXAMIC ACID** | **10,101** |
| **C** | **PLANKTON EXTRACT** | **6,734** |
| | WATER | 3,973 |
| | PLANKTON EXTRACT (scenedesmus obliquus extract) | 0,067 |
| | BUTYLENE GLYCOL | 2,693 |
| **C** | **SEA WATER** | **6,734** |
| **C** | **NIOSOME VITAMINE C** | **6,734** |
| | WATER | 2,053 |
| | 3-O-ETHYL ASCORBIC ACID | 0,269 |
| | MAGNOLOL | 0,134 |
| | HONOKIOL | 0,134 |
| | POLYGLYCERYL-6 LAURATE | 2,020 |
| | POLYGLYCERYL-10 OLEATE | 1,649 |
| | SORBITAN PALMITATE | 0,471 |
| **C** | **TETRA PAEPTIDES RIGIN** | **6,734** |
| | STEARETH-20 | 0,202 |
| | PALMITOYL TETRAPEPTIDE-7 | 0,003 |
| | GLYCERIN | 1,010 |
| | WATER | 5,498 |
| | CHLORHEXIDINE DIGLUCONATE | 0,013 |
| | POTASSIUM SORBATE | 0,006 |
| **B** | **BAKUCHIOL** | **6,734** |
| | BAKUCHIOL | 6,734 |
| | LINALOOL | 0.006 |
| **C** | **ASCORBYL GLUCOSIDE** | **3,367** |
| **B** | **TOCOPHERYL ACETATE** | **3,367** |
| **C** | **PANTHENOL** | **2,525** |
| | WATER | 0,774 |
| | PANTOLACTONE | 0,033 |
| | CITRIC ACID | 0 036 |
| **C** | **LACTOBACILLUS PLANTARUM FERMENT** | **3,367** |
| | MAL TODEXTRIN | 2,777 |
| | LACTOBACILLUS FERMENT | 0,589 |
| **C** | **GLUTATHIONE** | **2,020** |

The above composition was formed into a base product in accordance with the following method:
- Mix while cold phase A components under agitation,
- Add phase B component to form micelles,
- Blend and mix phase C component and add them into the blend previously obtained to form an emulsion.

### EXAMPLE 2: Evaluation of the trophic, energizing, anti-inflammatory and antioxidant efficacy of the composition of example 1

The purpose of the study described below is to assess in vitro the trophic, energizing, anti-inflammatory and antioxidant efficacy of the composition, by assessing its ability to stimulate the synthesis of collagen, glycosaminoglycans, filaggrin, ATP and to protect against pro-oxidant and pro-inflammatory damage agents on a model of human in vitro 3D reconstructed skin tissues.

In particular, the protocol provided the application of the composition in a single dose on the tissue surface for a 24-hour exposure. At the end of the experimental time, tissue homogenates and culture media were collected for the dosage of ATP, filaggrin, glycosaminoglycans and collagen.

The anti-inflammatory activity study was performed by dosing TNFalpha content (proinflammatory acute cytokine), by means of ELISA assay, after 24 and 48 hours from the inflammation induction (T0) and treatment with test item.

Finally, for the evaluation of the oxidative stress resistance, skin tissues were exposed to a pro-oxidant stress (H2O2) and to test item in repeated applications for a 72-hour experimental time. At the end of the treatment, cell viability was carried out by MTT assay.

### 1) Material and methods

### 1.1) Experimental model

The biological model used in the test was a highly differentiated 3D skin model consisting of normal, human-derived cells cultured on specially prepared tissue culture inserts. Tissues qualities are verified for the following parameters: normal histology (absence of significative alterations), cell viability (MTT OD > 0.8), barrier function integrity (4.00 < ET50 < 9.00 hrs), absence of bacteria, fungi, HIV, and Hepatitis B, C.

### 1.2) Preparation of the test composition and tissue exposure

All the tests were performed by using the tested composition applied on tissues surface at the concentration of 14,85%.

For the evaluation of the trophic and energizing activity study, tissues were treated for 24 hours with the test composition. At the end of experimental time, culture media were collected for Glycosaminoglycans, and Collagen dosages and tissue homogenates were collected for the dosage of Filaggrin and ATP.

In summary, the experimental protocol included:
- untreated tissues (negative control, CTR-);
- tissues treated with tested composition at the concentration of 14,85%.

For the anti-inflammatory activity study, tissues were treated with the sample and concomitantly exposed to Sodium Dodecyl Sulfate (SDS) at the concentration of 1% (10 mg/ml) to induce an acute inflammation for 24 and 48 hours. At the end of each experimental time, culture media were collected and TNF-alpha levels were measured by ELISA assay. The results were compared to negative control (tissue not exposed to inflammation stress, CTR-) and positive control (tissue exposed only to inflammation stress by SDS).

In particular, the experimental protocol included:
- untreated tissues (negative control, CTR-),
- inflamed and untreated tissues (positive control, CTR+)
- tissues inflamed and treated with tested composition at the concentration of 14,85%.

For the antioxidant study, a series of tissues was treated with the composition in presence of hydrogen peroxide (H2O2, 650 µM), typical oxidizing agent. Treatment was repeated every 24 hours for a total exposure time of 72 hours.

Protective efficacy is evaluated by evaluating tissues viability after the induction of stress in presence and in absence of the test composition.

In summary, the experimental protocol included:
- untreated tissues (negative control, CTR-),
- tissues treated with oxidizing agent H2O2 (positive control, CTR+),
- tissues treated with oxidizing agent H2O2 and tested composition at the concentration of 14,85%.

All the studies were performed in duplicate.

### 1.3) Trophic activity study

### Collagen assay:

The determination of collagen synthesis is carried out on culture medium by quantitative dye-binding method after a 24-hour treatment. The chromogen agent used in the assay is Sirius Red (Direct red 80). Sirius Red in an anionic dye with sulphonic acid side chain groups. These groups react with the side chain groups of the basic amino acids of collagen.

Collagen concentration (µg/well) is calculated by means of data interpolation on a standard curve obtained with known and increasing collagen concentrations. All assays were performed in triplicate.

### Filaggrin assay:

Filaggrin dosage in homogenate tissue at the end of the experimental treatments gives indication on the barrier function status of the tissues after product application.

Homogenated tissues of controls and tissues treated with tested composition were used for the dosage of filaggrin protein by means of ELISA method. Commercial kits were used for the determination.

The quantitative determination uses a calibration curve made-up of standard known and growing concentrations of standard protein. All assays were performed in triplicate.

### 1.4) Energyzing activity study

### ATP dosage

The determination of ATP synthesis was carried out on tissue homogenates by means of colorimetric method after a 24-hour treatment. A commercial kit was used for this purpose. ATP concentration is determined by phosphorylation of glycerol, resulting in a colorimetric (570 nm) product that is proportional to the amount of ATP present.

The quantitative determination uses a calibration curve made-up of known and growing concentrations of standard ATP. The results are expressed as ATP concentration in nmole/well. All assays were performed in triplicate.

### 1.5) Anti-inflammatory efficacy study

### TNF alpha dosage

In order to study the ability of the tested composition to inhibit the inflammatory response in vitro, in a system in which an inflammatory condition was induced, the production of the pro-inflammatory cytokine TNFalpha was assessed. At the end of treatments, culture media of each condition were collected for TNFalpha dosage using commercially available ELISA kits.

The % variation of the TNF-alpha content between the negative control and the sample or positive control was calculated, and the difference between these variations was used as index of the anti-inflammatory efficacy of the tested item. All assays were performed in triplicate.

### 1.6) Antioxidant efficacy study

Protective efficacy against a pro-oxidant agent is evaluated by means of the measure of tissues viability with MTT assay. The MTT (3,(4,5-dimethylthiazol-2)2,5 difeniltetrazolium bromide) test is a standard, simple and accurate colorimetric method for cell viability assessment. The assay is based on the intracellular reduction of the yellow tetrazolium salts by the mitochondrial enzyme succinate dehydrogenase in blue/purple formazan crystals. The reaction may therefore take place only in metabolically active cells and the value of the optical density obtained by photometric reading can be correlated to the number of viable cells.

At the end of experimental time, the tissues were rinsed with PBS (phosphate-buffered saline solution), stained with MTT solution 1 mg/mL and incubated for three hours at 36,5°C/5% CO2. Then the tissues were treated with isopropanol and incubated for two hours at room temperature. After isopropanol incubation, absorbance readings were performed at 570 nm by microplate reader (isopropanol was used as blank for reading). For each test condition the ratio of the average optical density of the treated cultures on the average optical density of negative controls determines the viability rate. The protection %, intended as cell viability % increase compared to the positive control condition, treated only with the damaging agent, is also calculated.

### 1.7) Statistical analysis

Obtained results were subjected to statistical analysis by means of Student test. Variations (vs CTR- e CTR+) are considered statistically significant with p<0.05.

### 2) Results

### 2.1 collagen synthesis

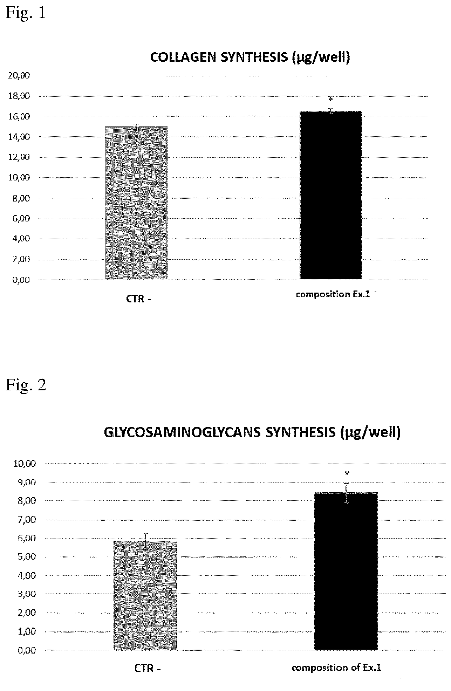

Results are given in Fig. 1 illustrating collagen content in not treated tissues (CTR-) and tissues treated with the composition of example 1 at the concentration of 14,85%. The results are expressed as mean value ± ST.DEV. of collagen content (expressed in µg/well) and. Significant variations (p<0.05) vs negative control are reported with asterisk (^{∗}).

The tissues treated with the composition according to example 1 showed a significant increase (10% (% variation compared to negative Control)) of collagen synthesis after a 24-hour treatment, indicating a trophic activity of the composition of the invention.

### 2.2 glycosaminoglycans synthesis

Results are given in Fig. 2 illustrating glycosaminoglycan content in not treated tissues (CTR- ) and tissues treated with the composition of example 1 at the concentration of 14,85%. The results are expressed as mean value ± ST.DEV. of glycosaminoglycan content (expressed in µg/well) and. Significant variations (p<0.05) vs negative control are reported with asterisk (^{∗}).

The tissues treated with the composition according to example 1 showed a significant increase (44% (% variation compared to negative Control)) of glycosaminoglycan synthesis after a 24-hour treatment, indicating a trophic activity of the composition of the invention.

### 2.3 filaggrin synthesis

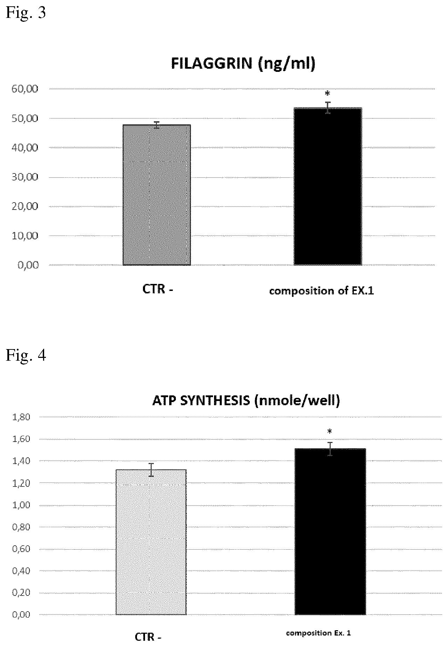

Results are given in Fig. 3 illustrating filaggrin content in not treated tissues (CTR-) and tissues treated with the composition of example 1 at the concentration of 14,85%. The results are expressed as mean value ± ST.DEV. of filaggrin content (expressed in µg/well) and. Significant variations (p<0.05) vs negative control are reported with asterisk (^{∗}).

The tissues treated with the composition according to example 1 showed a significant increase (12,1% (% variation compared to negative Control)) of filaggrin synthesis after a 24-hour treatment, indicating a trophic activity of the composition of the invention.

### 2.4 ATP synthesis

Results are given in Fig. 4 illustrating ATP synthesis in not treated tissues (CTR-) and tissues treated with the composition of example 1 at the concentration of 14,85%. The results are expressed as mean value ± ST.DEV. of ATP content (expressed in µg/well) and. Significant variations (p<0.05) vs negative control are reported with asterisk (^{∗}).

The tissues treated with the composition according to example 1 showed a significant increase (14,7 % (% variation compared to negative Control)) of ATP synthesis after a 24hour treatment, indicating a trophic activity of the composition of the invention.

### 2.5 Antioxidant activity

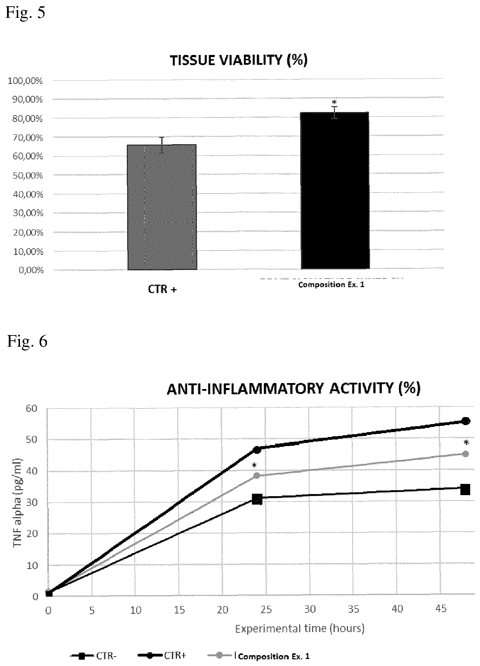

Results are given inf Fig. 5 illustrating tissue viability in each tested condition (CTR-, CTR+ and treated with the composition according to example 1 for 72 hours). The results are reported as mean value ± ST. DEV. (expressed in % vsCTR-). Mean % of protection compared to CTR+ is also determined. Significant variations (p<0.05) vs positive control are reported with asterisk (^{∗}).

The tissues treated with the damage agent showed a significant reduction of the cell viability (CTR+) while the tissues treated with the composition of example 1 showed a viability significantly higher 16,64% (% variation compared to the Control)) than CTR+ after a 72-hour repeated exposure to a pro-oxidant agent, indicating an antioxidant efficacy of the composition of the invention.

### 2.6 Anti-inflammatory efficacy

Results are given inf Fig.6 illustrating TNF alpha content in negative control (CTR-), positive control (CTR+) and tissues treated with the composition of example 1. The results are reported as mean value ± ST. DEV. (expressed in pg/ml) and mean % of the anti-inflammatory activity compared to CTR+ is determined. Significant variations (p<0.05) vs positive control are reported with asterisk (^{∗}).

TNF-alpha dosage analysis showed that the levels of pro-inflammatory cytokine released in tissues treated with the composition of example 1 are significantly lower than the one registered in CTR+ after 24 and 48 hours of treatment, indicating an anti-inflammatory efficacy of the composition according to the invention.

### 2.7 Conclusion

The composition according to the invention has shown a significant trophic efficacy by promoting the synthesis of collagen, glycosaminoglycans and filaggrin, and a significant energizing, antioxidant and anti-inflammatory capacity in an in vitro 3D reconstructed skin model.

### EXAMPLE 3: Evaluation of the capability of a composition according to the invention to modulate the skin dyschromia by using a model of in vitro reconstructed human pigmented epidermis

The aim is to evaluate the capability of the composition according to the invention to modulate the visibility of skin dyschromia by using a model of in vitro human reconstructed tanned epidermis.

### 1) Sample:

The sample is the composition according to example 1.

### 2) Materials and methods

### 2.1 Preparation of the test items and tissue exposure

The experimental model used in the test is Reconstructed Human Pigmented Epidermis (STERLAB, Batch 2111 TAN 01), from cells EP.DR P1 3 (keratinocytes) and ME.AI P2 14 (melanocytes).

Test item (composition Ex. 1) was tested at the concentration of 14,85%, by direct application on the epidermis. Particularly, 50 mg of tested product were applied on the epidermis surface.

The exposure timing was 6 days. At the end of each timing, the tissues were used for the determination of melanin content and for the image analysis to evaluate colour uniformity.

### 2.2 Melanin content dosage

At the end of each experimental time, the tissues were removed and plunged into 500 µl of Solvable (Perkin Elmer) at 100°C for 45 minutes. The optical density was measured at 450nm.

### 2.3 Image analysis

Some images were collected in the different experimental steps by means of a stereomicroscope (Zeiss, Tiesselab) at 30.0X magnification with associated camera (TiEsseLab PrimoCam 5 HD 5.0) on which an image analysis was performed using the Image J software to evaluate qualitatively the presence of pigmentation on the tissues.

### 2.4 Statistical analysis

All obtained data were subjected to statistical analysis by means of <T-test. Variations are considered significant for p<0.05.

### 3) Results

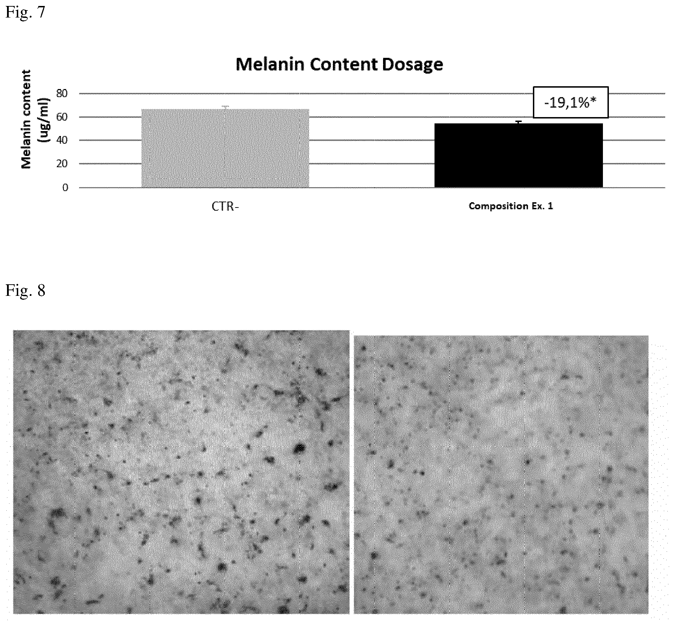

Data obtained for each tested series are reported in Fig. 7.

The results of the performed study show that the treatment of the pigmented tissues with the composition of example 1 significantly (p<0.05) reduces the melanin content in the considered biological model.

Some representative pictures of the appearance of the pigmented epidermis in the tissues used for the imaging evaluation of colour evenness, before and after the treatment with tested composition, are reported in Fig. 8.

The composition according to example 1 showed a visible and significant variation in the epidermis pigmentation with reduced melanin and greater uniformity in the pigment distribution.

### EXAMPLE 4: evaluation of cell proliferation capability

The proliferation test was carried out by using:
- Human skin fibroblasts
- Composition according to example 1 :0,01%; 0,005% and 0,0025% in culture medium
- Cell cultures without any treatment as negative control reference (CTR-) to calculate cell proliferation.

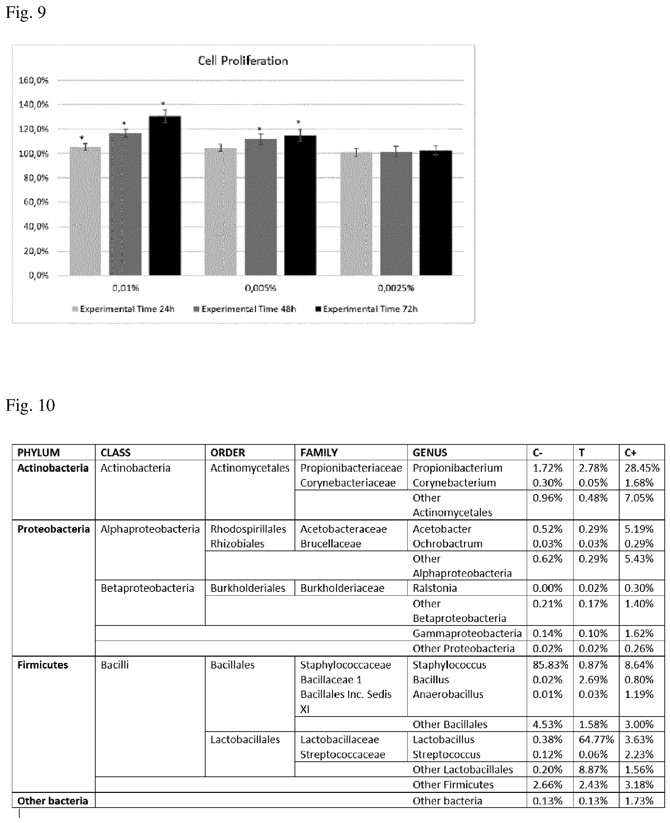

Fig. 9 illustrates cell proliferation study on cell culture (24H, 48H and 72 H) with the composition according to example 1 (3 concentrations).

The composition of example 1 enhances cells proliferation at each experimental time.

### EXAMPLE 5: Analysis of the capacity to maintain the balance of the skin microbiota

The aim of the study is to assess the ability of the composition according to the invention to prevent skin dysbiosis by helping in maintaining the balance of the skin microbiota on skin exposed to pollution. To reach this goal, in vitro reconstructed human skin tissues were inoculated with human skin microbiota from volunteers, then exposed to atmospheric pollutants (a solution of standard outdoor dust containing polluting particulate matter, National Institute of Standards & Technology, Standard Reference Material^{®} 1649b), and treated with the composition of Ex.1. Skin microbiota was collected in not treated tissues (negative controls), tissues exposed to pollutants (positive controls) and tissues treated with tested product (test) using swabs, and analysed by means of 16S rRNA analysis.

Experimental protocol provided:
- untreated epidermis+microbiota (CTR-);
- epidermis+microbiota treated with 50 µL of urban dust solution 100 mg/ml (CTR+);
- epidermis+microbiota treated with 50 µL of composition of example 1 and 50 µL of urban dust solution 100 mg/ml.

### 1) Materials and methods

### 1.1 test item

Composition according to example 1.

### 1.2 Experimental model

The biological model used in the test consists of three-dimensional reconstructed human epidermis, built from primary cultures of keratinocytes (EpiDerm - MatTek, batch 36107). The test system has an ultra-structure (tissue morphology and thickness) very similar to the human in vivo skin. All the tissue units were subjected to quality controls.

### 1.2 Microbiota inoculation

The microbiota was collected by swab from the face of two volunteers and pooled together. Then, the microbiota was used to inoculate in vitro reconstructed human skin tissues and incubated for two hours at 37°C.

### 1.3 Preparation of the test item and tissue exposure

Reconstructed human epidermis inoculated with microbiota was treated with product under study and with a solution of a standard urban dust containing typical environmental airborne pollutant for 24 hours. In particular, the dust powder is composed of selected polycyclic aromatic hydrocarbons (PAHs), nitro-substituted PAHs (nitro-PAHs), polychlorinated biphenyl (PCB) congeners, chlorinated pesticides and inorganic constituents in atmospheric particulate matter and similar matrices.

Epidermis was treated with 50 µL of test item at the concentration of 14,85% and then the environmental damage was induced for 24 hours.

### 1.4 Microbiome analysis

The skin microbiome analysis is based on the metagenomic analysis of the 16S rRNA gene. 16S rRNA gene was chosen since this gene is present in all bacterial (prokaryotic) cells genetic material, but not in human (eukaryotic) cells.

After a 24-hour treatment, the microbiota was collected by swab and processed for the microbiome analysis.

### 1.5 Results and statistics

The biodiversity index (Shannon index) and the statistics are calculated using a dedicate software (MicrobiomeAnalyst). Variations are considered statistically significant with p<0.05.

Table illustrated in Fig. 10 shows the taxa distribution in not treated tissues (C-), tissues exposed to pollutants (C+) and tissues treated with the composition ex.1 (T).

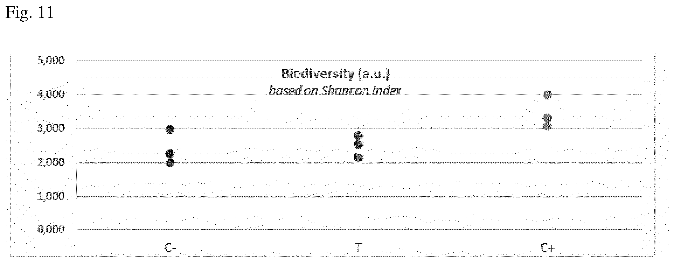

Fig 11 illustrates Shannon biodiversity of treated tissues (C-), tissues exposed to pollutants (C+) and tissues treated with tested composition ex.1 (T) obtained with MicrobiomeAnalyst analysis.

The analysis was based on the 10 most abundant genera and their parent taxa, which represent between 75% and 100% of the inoculated microbiotas.

The 10 most abundant genera across analysed samples were: two Actinobacteria (Propionibacterium and Corynebacterium), two Alphaproteobacteria (Acetobacter and Ochrobactrum), one Betaproteobacteria (Ralstonia), three Bacillales (Staphylococcus, Bacillus, and Anaerobacillus) and two Lactobacillales (Lactobacillus and Streptococcus).

Compared to the negative control, the pollution treatment (C+) resulted in an increase of opportunistic pathogens, such as Propionibacterium and Streptococcus. Treatment with the tested composition according to example 1 (T) was able to inhibit the increase of these pathogens, thus suggesting an efficacy of the active in maintaining the balance of the skin microbiota on skin exposed to pollution.

This is confirmed also by the Shannon biodiversity analysis (Fig. 11) that indicates how T treated samples are more similar to C- than C+.

Interestingly, Lactobacillus, considered beneficial for skin health, is present in high % after treatment with the composition ex.1. This data could be due to an eutrophic activity of the product or could be the consequence of Lactobacillus DNA presence in the product.

### EXAMPLE 6: Evaluation of repairing effect

The aim of this study is the determination of the in vitro capability of the composition according to the invention to promote wound healing, induced in an in vitro experimental model represented by human skin Keratinocytes in monolayer. The repairing efficacy was assessed by the product ability to promote the healing of damaged tissue in the in vitro system.

### 1) Materials and method

### 1.1 Monitored experimental conditions

The test was carried out in triplicate by using:
- cell cultures in which the composition of ex.1 was added after the wound creation to study the regeneration effect - Comp ex.1;
- cell cultures without any treatment - UNTREATED CONTROL, CTR-.

### 1.2 Artificial wound creation

Cell cultures of human Keratinocytes were used. The cells were put in well-plate filled with Dulbecco's modification of Eagle medium (DMEM) supplemented with Foetal Bovine Serum 10%. Cells were incubated at 37°C and 5% CO2 until full confluence.

An artificial wound was mechanically created in the cell monolayer by sliding the tip of a pipette on the cell monolayer. For each experimental condition, 3 replica were performed.

### 1.3 Monitored experimental times

The repairing effect of the test item was determined by acquisition of photographic images of the cell cultures at T0, T2H, T8H and T24H after the wound creation.

### 1.4 Wound repairing evaluation

At each experimental time, cells were visually checked using (OPTIKA, XDS-2) at 10X and pictures with camera (TiEsseLab PrimoCam HD 5.0 with sensor Micron MT9P001) were collected. Pictures were analysed with Image J software.

The distances between the wound margins for each picture were measured in µm and were recorded in 3 points of the wound length. The variation on wound margins were analysed by T-test. Some representative images of the wound healing progress are reported for the different experimental conditions at all the monitored times.

### 2) Results

The data in table 3 represent the distance (expressed as mean value ± standard deviation) between the wound margins (µm).

The data in table 3 represent the percentage variation of the wound margin distance of treated cells and untreated wells for each experimental time compared to T0.

In the tables 4 and 5, the results on the statistical analysis were reported (T test).

In the final part of this paragraph, some series of pictures kept during the test were reported.

**Table 3. Reported values (expressed by mean ± stand deviation.) refer to the distance between the wound margins (µm), recorded at the different experimental times.**

| | **CR-** | | **Comp ex.1 0,01%** | | **Comp ex.1 0,005 %** | | **Comp ex.1 0,0025 %** | |
|---|---|---|---|---|---|---|---|---|
| | Mean value (µm) | St.dev. | Mean value (µm) | St.dev. | Mean value (µm) | St.dev. | Mean value (µm) | St.dev. |
| **T 0H** | 1419,9 | 49,8 | 1470,2 | 52,7 | 1443,3 | 76,1 | 1392,8 | 34,2 |
| **T 2H** | 1386,6 | 78,7 | 1389,9 | 30,0 | 1377,1 | 63,4 | 1353,1 | 29,4 |
| **T 8H** | 1282,0 | 68,9 | 1222,6 | 17,8 | 1260,4 | 42,8 | 1238,8 | 65,6 |
| **T 24H** | 997,9 | 94,8 | 854,2 | 41,0 | 927,6 | 40,2 | 967,7 | 55,9 |

**Table 4. Reported values refer to the % reduction of the distance of the wound margins artificially inducted for each experimental time compared to T0.**

| | **CTR- vs TO** | **Comp ex.1** | **Comp ex.1** | **Comp ex.1** |
|---|---|---|---|---|
| | | **0,01% vs T0** | **0,005 % vs T0** | **0,0025 % vs T0** |
| **T 2H** | 2,3 % | 5,5 % | 4,6 % | 2,8 % |
| **T 8H** | 9,7 % | 16,8 % | 12,7 % | 11,1 % |
| **T 24H** | 29,7 % | 41,9 % | 35,7 % | 30,5 % |

**Table 5: statistical analysis versus TO**

| | **CR-** | | **Comp ex.1 0,01%** | | **Comp ex.1 0,005 %** | | **Comp ex.1 0,0025 %** | |
|---|---|---|---|---|---|---|---|---|
| | VAR vs TO | T.TEST | VAR vs TO | T.TEST | VAR vs TO | T.TEST | VAR vs TO | T.TEST |
| **T 2H** | -2,3 % | 0,296 | -5,5 % | 0,006 | -4,6 % | 0,004 | -2,8 % | 0,004 |
| **T 8H** | -9,7 % | 0,000 | -16,8% | 0,000 | -12,7 % | 0,000 | -11,1 % | 0,000 |
| **T24** | -29,7 % | 0,000 | -41,9% | 0,000 | -35,7 % | 0,000 | -30,5 % | 0,000 |

**Table 6: Statistical analysis (p-value) versus CTR-**

| | **Comp ex.1** | **Comp ex.1** | **Comp ex.1** |
|---|---|---|---|
| | **0,01% vs CTR-** | **0,005 % vs CTR-** | **0,0025 % vs CTR-** |
| **T 0H** | 0,054 | 0,450 | 0,197 |
| **T 2H** | 0,907 | 0,783 | 0,250 |
| **T 8H** | 0,023 ^{∗} | 0,437 | 0,192 |
| **T 24H** | 0,001 ^{∗} | 0,057 | 0,422 |

The obtained results show that the treatment of cell cultures with the composition of example 1 significantly promotes wound repair by making it faster.

According to the obtained kinetics, repairing process in cells treated with the product was significantly more efficient compared to untreated condition after 8 and 24 hours of treatment.

### EXAMPLE 7 Cosmetic product including a composition according to the invention

The following composition is prepared from the ingredients listed in Table 7 below. The percentages are weight percentages.

**Table 7**

| **INGREDIENTS** | ***INCI UE*** | **%** |
|---|---|---|
| EAU PURIFIEE | AQUA | 41,836500 |
| SEPIMAX ZEN | POLYACRYLATE | 0,500000 |
| | CROSSPOLYMER-6 | |
| | t-BUTYL ALCOHOL | |
| | AQUA | |
| 1,3-Propanediol 05 | PROP ANEDIOL | 5,500000 |
| MINACARE PENTIOL GREEN / + A-LEEN 5 | PENTYLENE GLYCOL | 2,000000 |
| AMAZE XT | DEHYDROXANTHAN GUM | 0,500000 |
| | AQUA | |
| HU MACADAMIA BIO | MACADAMIA | 5,000000 |
| | INTEGRIFOLIA SEED OIL | |
| NOVATEXT SOFT FOCUS 3 | HYDROGENATED | 10,000000 |
| | VEGETABLE OIL | |
| | SILICA | |
| INST'TIGHT | AQUA | 2,000000 |
| | GLYCERIN | |
| | GLEDITSIA TRIACANTHOS | |
| | SEED EXTRACT | |
| | CAPRYLYL GLYCOL | |
| BIO-OSLP | GLYCYRRHIZA GLABRA | 0,010000 |
| | ROOT EXTRACT | |
| BRIGHTENYL | GLYCERIN | 1,500000 |
| | AQUA | |
| | DIGLUCOSYL GALLIC ACID | |
| MEGASSANE | CAPRYLIC/CAPRIC | 0,300000 |
| | GLYCERIDES | |
| | PHAEODACTYLUM | |
| | TRICORNUTUM EXTRACT | |
| | TOCOPHEROL | |
| AQUAXYL | XYLITYLGLUCOSIDE | 1,500000 |
| | ANHYDROXYLITOL | |
| | AQUA | |
| | XYLITOL | |
| | GLUCOSE | |
| PHYTOCELLTEC | ISOMALT | 0,500000 |
| NUNATAK | AQUA | |
| | LECITHIN | |
| | SAPONARIA PUMILA | |
| | CALLUS CULTURE | |
| | EXTRACT | |
| HYDRASENSYL GLUCANS | AQUA | 2,500000 |
| | PENTYLENE GLYCOL | |
| | BETA-GLUCAN | |
| | CAPRYLYL GLYCOL | |
| REVINAGE WPO | ASTROCARYUM | 2,000000 |
| | MURUMURU SEED BUTTER | |
| | GOSSYPIUM HERBACEUM | |
| | SEED OIL | |
| | BIDENS PILOSA EXTRACT | |
| | LINUM USIT A TISSIMUM | |
| | SEED OIL | |
| | TOCOPHEROL | |
| ARGIRELINE POUDRE | ACETYL HEXAPEPTIDE-8 | 0,002500 |
| AKOACTIVE DRAGON | CAPRYLIC/CAPRIC | 1,000000 |
| | TRIGLYCERIDE | |
| | 4-BUTYLRESORCINOL | |
| | PAEONIA OFFICINALIS | |
| | FLOWER EXTRACT | |
| | PEG-7 GLYCERYL | |
| | COCOATE | |
| ACTIBIOME GPA | GLYCERIN | 0,500000 |
| | AQUA | |
| | MARIS AQUA | |
| | PHENETHYL ALCOHOL | |
| | LAMINARIA DIGITATA | |
| | EXTRACT | |
| | CHLORELLA VULGARIS | |
| | EXTRACT | |
| | SACCHARIDE ISOMERATE | |
| ALPHA-MELIGHT ECO | BISABOLOL | 1,000000 |
| **COMPOSITION OF EXAMPLE 1** | NIACINAMIDE | 14,851000 |
| | GLYCERIN | |
| | AQUA | |
| | TRANEXAMIC ACID | |
| | BUTYLENE GLYCOL | |
| | BAKUCHIOL | |
| | MARIS AQUA | |
| | ASCORBYL GLUCOSIDE | |
| | TOCOPHERYL ACETATE | |
| | MAL TODEXTRIN | |
| | PANTHENOL | |
| | GLUT A THIONE | |
| | POLYGLYCERYL-6 | |
| | LAURATE | |
| | POLYGLYCERYL-10 | |
| | OLEATE | |
| | GLYCERYLAMIDOETHYL | |
| | METHACRYLA TE/STEARYL | |
| | METHACRYLATE | |
| | COPOLYMER | |
| | LACTOBACILLUS | |
| | FERMENT | |
| | SORBITAN PALMITATE | |
| | CERAMIDE NP | |
| | 3-O-ETHYL ASCORBIC | |
| | ACID | |
| | STEARETH-20 | |
| | MAGNOLOL | |
| | HONOKIOL | |
| | PLANKTON EXTRACT | |
| | PANTOLACTONE | |
| | CITRIC ACID | |
| | CHLORHEXIDINE | |
| | DIGLUCONATE | |
| | LINALOOL | |
| | POTASSIUM SORBATE | |
| | PALMITOYL | |
| | TETRAPEPTIDE-7 | |
| ASCORBYL GLUCOSIDE | ASCORBYL GLUCOSIDE | 0,500000 |
| COPHEROL 1250 C | TOCOPHERYL ACETATE | 1,000000 |
| DERMOSOFT 1388 ECO pH 5.5 | AQUA | 4,000000 |
| | GLYCERIN | |
| | SODIUM LEVULINATE | |
| | SODIUM ANISATE | |
| AMIHOPE LL | LAUROYL LYSINE | 0,500000 |
| LOW LUSTER | MICA | 1,000000 |
| PIGMENT RONAFLAIR | CI 77120 | |
| | CI 77891 | |
| | | **100,000000** |

## Claims

1. Composition, **characterised in that** it is provided in the form of an aqueous dispersion of spherules composed of at least one lipids bilayer encapsulating aqueous phase comprising at least one active agent, the lipids bilayer being composed of a mixture of liposoluble compounds comprising at least one (glycerylamidoethyl methacrylate and stearyl methacrylate) copolymer and at least one type III Ceramide and eventually at least one other liposoluble compound.

2. Composition according to any one of claims 1 to 3, **characterised in that** it comprises between 1 to 30 %, by weight, of (glycerylamidoethyl methacrylate and stearyl methacrylate) copolymers and between 0,1 to 5% of type III Ceramides, by weight relative to the total weight of the composition.

3. Composition according to claim 1, **characterised in that** it comprises at least one another liposoluble compound which is bakuchiol or/and α-tocopherylacetate, preferably bakuchiol and α-tocopherylacetate.

4. Composition according to claim 1 or 2, **characterised in that** it comprises at least 1%, preferably at least 10%, more preferably at least 20%, more preferably at least 30%, of spherules, by weight relative to the total weight of the composition.

5. Composition according to any one of claims 2 to 4, **characterised in that** it comprises between 1 to 10 % of bakuchiol and between 1 to 10 % of α-tocopheryl acetate, by weight relative to the total weight of the composition.

6. Composition according to any one of claims 1 to 5, **characterised in that** the aqueous phase encapsulated in the spherules comprises at least one active agent selected from seawater, Scenedesmus obliquus extract, tranexamic acid, L-glutathione, niacinamide, D-panthenol, tetrapeptide N-Palmitoyl-Gly-Gln-Pro-Arg, probiotic microorganisms of the specie lactobacillus plantarum, ascorbyl glucoside, niosomes of C vitamin, seawater or any one of their combinations.

7. Composition according to claim 6, **characterised in that** the encapsulated aqueous phase comprises tranexamic acid, L-glutathione, ascorbyl glucoside, niacinamide, seawater and D-panthenol.

8. Composition according to claim 6, **characterised in that** the encapsulated aqueous phase comprises Scenedesmus obliquus extract, tranexamic acid, L-glutathione, niacinamide, D-panthenol, tetrapeptide N-Palmitoyl-Gly-Gln-Pro-Arg, probiotic microorganisms of the specie lactobacillus plantarum, ascorbyl glucoside, niosomes of C vitamin and seawater.

9. Composition according to anyone of claims 8, **characterised in that** it comprises, by weight relative to the total weight of the composition:
- between 1 to 30 % of glycerylamidoethyl methacrylate and stearyl methacrylate copolymer,
- between 0,1 to 5% of at least one type III Ceramides,
- between 1 to 10 % of bakuchiol,
- between 1 to 10 % of α-tocopherylacetate,
- between 1 to 15 % of tranexamic acid,
- between 1 to 15 % of ascorbyl glucoside,
- between 1 to 10 % of seawater,
- between 1 to 5 % of D-panthenol,
- between 1 to 5 % of probiotic microorganisms of the specie lactobacillus plantarum,
- between 1 to 5 % of L-glutathione,
- between 1 to 10 % tetrapeptide N-Palmitoyl-Gly-Gln-Pro-Arg,
- between 1 to 25 % of niacinamide,
- between 1 to 10 % of Scenedesmus obliquus extract,
- between 1 to 10% of niosomes of C vitamin.

10. A process for preparing a composition according to any one of claims 1 to 9, **characterised in that** it comprises a first step of mixing (glycerylamidoethyl methacrylate and stearyl methacrylate) copolymers with type III Ceramides, and eventually bakuchiol or/and α-tocopherylacetate, and a second step of adding an aqueous soution comprising at least one active agent selected from Scenedesmus obliquus extract, tranexamic acid, L-glutathione, niacinamide, D-panthenol, tetrapeptide N-Palmitoyl-Gly-Gln-Pro-Arg, probiotic microorganism of the species lactobacillus plantarum, ascorbyl glucoside, niosomes of C vitamin, seawater or anyone of their combination.

11. Cosmetic or dermatologic product, **characterised in that** it comprises, by weight relative to the total weight of the product, between 0, 5 to 15 % of a composition as defined in any one of claims 1 to 9, preferentially between 10 to 15%.

12. Process for the cosmetic treatment of a healthy skin, **characterised in that** it consists in applying to the healthy skin a sufficient amount of a composition according to any one of claims 1 to 9 or a product according to claim 11.

13. Cosmetic use of a composition according to any one of claims 1 to 9, or of a product according to claim 11, for treating or preventing, in a healthy skin, skin ageing or photo-ageing, for moisturizing the skin, for reinforcing the lipid barrier of the epidermis, for restoring or maintaining the integrity of the stratum corneum, for maintaining, stimulating or restoring cutaneous homeostasis, in particular the epidermis homeostasis, for maintaining stimulating, restoring or stimulating skin trophic activity, in particular for stimulating collagen and/or glycosaminoglycan and /or filaggrin synthesis, for stimulating the cellular energy metabolism, in particular for improving ATP synthesis and cell metabolism, for reducing Reactive Oxygen Species levels naturally occurring in skin cells, for maintaining, stimulating or restoring cell regeneration, for diminishing the appearance of wrinkles, supporting natural barrier function, or for lightening, whitening or depigmenting the skin, in particular pigment spots or dark circles under the eyes, the cosmetic use being apply to an healthy skin.

14. Composition according to any one of claims 1 to 9, or product according to claim 11, for its use for preventing, reducing or treating skin inflammatory, for maintaining the skin microbiota balance, in particular in case of acne, for restoring skin after dermatological or chirurgical treatment or for restoring atopic skin, in particular for restoring cell regeneration, cell homeostasis, cellular energy metabolism and the integrity of the stratum corneum after such treatments or in atopic skin, for reducing oxidative stress in skin cells, in particular due to environmental factors like urban or digital pollution or UV, for reducing or treating skin dyschromia due to dermatologic diseases, for wound healing.

15. Delivery system for active agents **characterized in that** it comprises an aqueous dispersion of spherules composed of at least one lipids bilayer encapsulating aqueous phase, the lipids bilayer being composed of a mixture of liposoluble compounds comprising at least one (glycerylamidoethyl methacrylate and stearyl methacrylate) copolymer and at least one type III Ceramide and eventually at least one other liposoluble compound.

16. Delivery system according to claim 15, **characterised in that** it comprises at least one another liposoluble compound which is bakuchiol or/and α-tocopherylacetate, preferably bakuchiol and α-tocopherylacetate.

17. Use of a composition according to any one of claims 1 to 9, or of a delivery system according to claim 15, to generate an osmotic gradient and to enhance skin penetration.
